Europäisches Patentamt

European Patent Office

Office européen des brevets

(19)

(11) Publication number: 0 140 398
A1

(12) EUROPEAN PATENT APPLICATION

(21) Application number: 84201132.2

(22) Date of filing: 02.08.84

(51) Int. Cl.⁴: **A 61 K 9/48**
A 61 K 31/135

(30) Priority: 10.08.83 NL 8302806

(43) Date of publication of application:
08.05.85 Bulletin 85/19

(84) Designated Contracting States:
AT BE CH DE FR GB IT LI LU NL SE

(71) Applicant: DUPHAR INTERNATIONAL RESEARCH B.V
C.J. van Houtenlaan 36
NL-1381 CP Weesp(NL)

(72) Inventor: Wilson, Johannes Hendrikus Gerardus
c/o OCTROOIBUREAU ZOAN B.V. Apollolaan 151
NL-2077 AR Amsterdam(NL)

(74) Representative: Muis, Maarten et al,
OCTROOIBUREAU ZOAN B.V. Apollolaan 151
NL-1077 AR Amsterdam(NL)

(54) Method of orally administering a composition to horses.

(57) The invention relates to a method of administering to horses medicaments and/or other substances which promote the welfare of these animals by presenting them incorporated in capsules so that they are taken freely.

The method is suitable for example for the treatment of podotrochlosis by presenting the horses capsules with isoxsuprine or a salt thereof.

EP 0 140 398 A1

Croydon Printing Company Ltd.

Method of orally administering a composition to horses.

The invention relates to a method of orally administering a composition to horses.

It is known that the oral administration of medicaments and other substances which promote the welfare of horses is particularly troublesome. Horses are very critical as regards that which they take in orally. The oral administration to horses of substances having a certain smell and/or taste is substantially impossible. Such compositons are nearly alway refused by the animals.

According to the invention described in German Offenlegungsschrift 2404007, this problem has been solved by bringing the compositions or substances to be administered in the form of a solid or doughy mass soluble in the saliva, or dispensing them in cartridges or capsules and connecting the substances in this form to or in the snaffle or bit before this is placed in the horse's mouth.

This method suffers from the disadvantage of being rather cumbersome, requiring a special snaffle or bit having recesses, and being not suitable for all compositions and substances.

It has surprisingly been found that horses accept and consume gelatin capsules without any problem. All this was found during an investigation into the influence of p-hydroxy-$\alpha$-[1-{(1-methyl-2-phenoxyethyl)-amino}ethyl]benzyl alcohol (known under the tradename isoxsuprine) on navicular disease in horses (podotrochlosis). Since isoxsuprine is a vasodilatory agent it might be active against the said disease the cause of which is presumably an insufficient blood circulation in the lower legs of the horse.

A commercially available composition for this indication which contains isoxsuprine is a paste to be administered orally. It will be obvious that such an oral administration to horses is an action which is difficult to perform.

During the investigation to come to a composition which is easier to administer it was found that the retard formulation of isoxsuprine known for human application, i.e. isoxsuprine polystyrene sulphonate in capsules, is taken without any problem by horses when such capsules are or are not mixed with some food.

On the basis of the fact that it is generally known that it is very difficult to administer anything orally to horses, it may be said to be exceptionally surprising that the administration of capsules presents no problem whatsoever.

In six institutions in England, the Netherlands and Germany experiments are now being performed with the capsules in question. Nowhere were any problems encountered in the administration, which was said to be very surprising by those skilled in the art.

Other compounds which can be administered by means of capsules to horse are vitamin preparations such as vitamin A palmitate, riboflavine-5-phospate sodium, calcium pantothenate, thiamine mononitrate, nicotinic acid and nicotinic acid amide.

CLAIMS:

1. A method of orally administering to horses medicaments or other substances which promote the welfare of horses, characterized in that the agents are taken freely by the horses by presenting them while incorporated in capsules.

2. A method as claimed in Claim 1, characterized in that isoxsuprine, or a salt thereof, as such or mixed with auxiliary substances, is made to be taken freely by the horses for the treatment of podotrochlosis by presenting it while incorporated in capsules.

3. A method as claimed in Claim 2, characterized in that isoxsuprine polystyrene sulphonate is made to be taken by the horses by presenting it while incorporated in capsules.

**PARTIAL EUROPEAN SEARCH REPORT**
which under Rule 45 of the European Patent Convention
shall be considered, for the purposes of subsequent
proceedings, as the European search report

EP 84 20 1132

## DOCUMENTS CONSIDERED TO BE RELEVANT

| Category | — Citation of document with indication, where appropriate, of relevant passages | Relevant to claim | CLASSIFICATION OF THE APPLICATION (Int. Cl 4) |
|---|---|---|---|
| X | AUSTRALIAN VETERINARY PRACTITIONER vol. 9, no. 2, June 1979 R.J. ROSE et al.: "Some Effects of Oral Chlorbutol in the Horse", pages 121-122 <br><br> * Page 121, sub. "Summary" * | 1-3 | A 61 K 9/48 <br> A 61 K 31/135 |
| Y | EQUINE VETERINARY JOURNAL, vol. 15 no. 3, 1983 R.J. ROSE et al.: "Studies on isoxsuprine hydrochloride for the treatment of navicular disease" pages 238-243 <br><br> * Page 238, left-hand column, sub. "Summary" to last paragraph * | 1,2 | |
| Y | UNLISTED DRUGS, vol. 26, no. 3, March 1974, page 38g ./. | | |

**TECHNICAL FIELDS SEARCHED (Int. Cl.4)**

A 61 K 9/00
A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely:

Claims searched incompletely: 1-3

Claims not searched:

Reason for the limitation of the search: Method for treatment of the human or animal body by surgery or therapy (see art. 52(4) of the European Patent Convention).

| Place of search | Date of completion of the search | Examiner |
|---|---|---|
| The Hague | 19-11-1984 | WILLEKENS |

EPO Form 1505.1. 03.82

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| | * Page 38g: Defencin * | 1-3 | |
| | -- | | |
| PX | DE - A - 3 317 280 (MEDICAL RESEARCH) | | |
| | * Claims; page 8, lines 6-23 * | 1-3 | |
| | ---- | | |

**TECHNICAL FIELDS SEARCHED (Int. Cl.4)**